# EUROPEAN PATENT SPECIFICATION

(11) **EP 4 074 733 B1**
(45) Date of publication and mention of the grant of the patent: **08.07.2026**
(21) Application number: 19954766.2
(22) Date of filing: 10.12.2019
(51) Int. Cl.: A61K 38/00, C07K 14/31, C07K 14/315, A61P 37/02

(54) **IMMUNOGLOBULIN BINDING PROTEIN, PREPARATION METHOD AND APPLICATION THEREOF**
IMMUNGLOBULINBINDENDES PROTEIN, VERFAHREN ZU SEINER HERSTELLUNG UND VERWENDUNG DAVON
COMPOSITION DE LIAISON D'IMMUNOGLOBULINE, SON PROCÉDÉ DE PRÉPARATION ET SON APPLICATION

(30) Priority: 06.12.2019 CN 201911241119
(43) Date of publication of application: 19.10.2022
(73) Proprietor: Guangzhou Koncen Bioscience Co., Ltd., Guangzhou, Guangdong 510000 (CN)
(72) Inventor: ZHANG, Haizhen, Guangzhou, Guangdong 510000 (CN); YANG, Zhenggen, Guangzhou, Guangdong 510000 (CN); YU, Boguang, Guangzhou, Guangdong 510000 (CN); WANG, Yunxi, Guangzhou, Guangdong 510000 (CN); LIN, Dahong, Guangzhou, Guangdong 510000 (CN); LUO, Lihua, Guangzhou, Guangdong 510000 (CN); CHEN, Xiaoyuan, Guangzhou, Guangdong 510000 (CN)
(74) Representative: Scholl, Matthias
(86) International application number: PCT/CN2019/124292
(87) International publication number: WO 2021/109177

(56) References cited:
- WO-A1-2015/005859
- CN-A- 1 634 990
- CN-A- 1 642 976
- CN-A- 102 365 361
- CN-A- 103 184 229
- CN-A- 103 748 221
- CN-A- 104 540 853
- CN-A- 105 481 954
- YANG HUA ET AL: "Evolutional selection of a combinatorial phage library displaying randomly-rearranged various single domains of immunoglobulin (Ig)-binding proteins (IBPs) with four kinds of Ig molecules", BMC MICROBIOLOGY, BIOMED CENTRAL LTD, GB, vol. 8, no. 1, 13 August 2008 (2008-08-13), pages 137, XP021042350, ISSN: 1471-2180, DOI: 10.1186/1471-2180-8-137
- SUSANNE GÜLICH ET AL: "Engineering streptococcal protein G for increased alkaline stability", PROTEIN ENGINEERING, 1 October 2002 (2002-10-01), England, pages 835 - 842, XP055556696, Retrieved from the Internet <URL:https://academic.oup.com/peds/article-pdf/15/10/835/18546200/150835.pdf> DOI: 10.1093/protein/15.10.835
- "Protein Purification", 20 January 2012, INTECH, ISBN: 978-953-30-7831-1, article TOVE BOSTRM ET AL: "Purification Systems Based on Bacterial Surface Proteins", XP055243910, DOI: 10.5772/31078

## Description

The disclosure belongs to the field of biomedical materials and blood purification, and more particularly to an immunoglobulin binding protein and a preparation method and application thereof.

Autoimmune diseases are a series of symptoms and diseases caused by damage to tissues and organs of the whole body due to variation of autoantibodies, and are one of main diseases which are difficult to treat and threaten human health and life, and so far there is no effective treatment. At present, the main method for treating the autoimmune diseases is to apply immunosuppressive medicaments and plasma replacement. The immunosuppressive medicaments inhibit the immune system of the body and reduce the damage of the antibodies in the body to the tissues and the organs so as to achieve the purpose of relieving the symptoms and delaying the disease progression. The immunosuppressive medicaments inhibit the generation of all the antibodies of the body, have large toxic and side effects, and have no ideal treatment effect on a plurality of autoimmune diseases and symptoms thereof. The plasma replacement is to replace plasma in the body of a patient with normal human plasma periodically so as to remove the variant immunoglobulin in the body. The transfusion of allogeneic plasma possibly causes: 1) cross infection, 2) allergic reaction, 3) bleeding tendency, etc. Therefore, seeking better treatments for autoimmune diseases is a major research topic in the medical field.

Protein A immunoadsorption is that natural or recombinant protein A is coupled to a vector through a covalent bond to synthesize an immunoadsorbent, and immunoglobulin in blood can be effectively removed through an extracorporeal circulation method. Commercial protein A immunoadsorption columns Immunosorba and Prosorba (both are products of German Fresenius Company) have been approved by the U.S. Food and Drug Administration (FDA) in 1999-2001 in succession for treating idiopathic thrombocytopenic purpura and rheumatoid arthritis (Prosorba) and hemophilia (Immunosorba) with inhibitory factors. A new adsorption column LIGASORB was launched by Fresenius in 2015 for treating acute autoimmune diseases. Its ligand is the protein A recombined through genetic engineering, its vector is polymethacrylate, and the new adsorption column LIGASORB is disposable. Clinical application results show that the protein A immunoadsorption blood purification treatment based on the affinity chromatography principle has following unique treatment effects on autoimmune diseases: 1) the treatment effects on the autoimmune diseases with non-ideal treatment effects by a plurality of immunosuppressive drugs are better; 2) it has almost no toxic or side effects on the body; 3) the side effects of plasma replacement are overcome; and 4) it is applied together with the immunosuppressive drugs, so that the dosage of the immunosuppressive drugs can be minimized to reduce the toxic or side effects of the immunosuppressive drugs, etc. With the rapid development of medicine, the application of organ transplantation is more and more common. The functions of the protein A immunoadsorption blood purification treatment are more and more important in the organ transplantation, and the application is more and more extensive: 1) the probability of rejection reaction can be reduced by performing the protein A immunoadsorption blood purification treatment before the organ transplantation; 2) during organ matching, the matching problem caused by inconsistent ABO blood types can be eliminated by applying the protein A immunoadsorption blood purification treatment; 3) when the sign of the rejection reaction occurs, the occurrence of the rejection reaction can be prevented by applying the protein A immunoadsorption blood purification treatment; and 4) after the organ transplantation, the dosage of the immunosuppressive drugs can be reduced by applying the protein A immunoadsorption blood purification treatment, so that the toxic or side effects of the immunosuppressive drugs, etc. are reduced. Meanwhile, more and more studies show that the protein A immunoadsorption blood purification treatment also has a good adjuvant treatment effect on malignant tumors, and the chemotherapy and radiotherapy effects of the malignant tumors can be enhanced because immunosuppressive compounds generated in the bodies of tumor patients are eliminated by immunoadsorption.

The Immunoglobulin binding proteins (IBP) are protein molecules which are derived from bacteria and can selectively adsorb immunoglobulins of mammals (including human). These molecules often contain a certain degree of repetitive sequence (namely homologous fragments), have simple structure and high stability, and can be reversibly bound to the immunoglobulins, etc. The most representative three IBPs are protein A, protein G and protein L.

Protein A with full name of Staphylococcal protein A (SPA) is the immunoglobulin binding protein that is studied most thoroughly so far. The molecular weight of the natural protein A is about 42 kDa, the protein A is bound to the cell wall of bacteria through cell wall binding domain at its carboxyl terminal and consists of five immunoglobulin binding regions called as regions E, D, A, B and C, and each protein A molecule can be bound with two IgG molecules. The reactivity of the protein A with three subtypes of IgG1, IgG2 and IgG4 of human IgG is 100%, and the reactivity of the protein A with IgG3 is low and is about 35%. The reactivity of the protein A with IgM and IgA is relatively low, and the protein A is almost non-reactive to IgE.

Protein G with full name of Streptococcal protein G (SPG) is derived from the cell surface of another pathogenic gram-positive bacterium-streptococcus. The molecular weight of the natural protein G is about 65 kd, and the protein G contains three IgG binding structural domains C1, C2 and C3 with extremely high homology. Different from protein A, the protein G binds to all subclasses of human IgG, the reactivity of the protein G is 100%, and the protein G does not bind IgA, IgM, IgD and IgE.

Protein L has a full name of protein L from peptostreptococcus magnus (PPL). The natural protein L is derived from the cell wall of the peptostreptococcus magnus. The binding of the protein A and the protein G to human Ig mainly derives from the binding to an Fc segment, namely Ig heavy chain, so the binding of the two IBP molecules and the human Ig is limited by the classification of the Ig (different types of Ig, namely IgG, IgA, IgM, IgE and IgD are distinguished due to different antigenicity of the heavy chain). The binding of the protein L to the human Ig mainly derives from the binding to an Ig light chain, so the binding of the IBP molecules and the human Ig is not limited by the classification of the Ig but limited by the classification (the human Ig is divided into a κ type and a λ type according to different antigenicity of the light chain). The molecular weight of the protein L is 76 KD, consisting of five Ig binding structural domains, namely B1, B2, B3, B4 and B5. The protein L can bind to three subtypes, namely κI, κIII and κIV in the κ type Ig, but does not bind κII subtype and the λ type. The affinity of the protein L to the IgG, the IgM and the IgA is basically consistent. According to the description, the binding characteristics of the protein A, the protein G and the protein L are different or have certain complementarity, so if the protein A, the protein G and the protein L can be comprehensively utilized and have complementary advantages, it is expected to develop multifunctional IBP molecules with wider reactivity and higher affinity.

CN 1634990 A discloses the construction of Ig binding molecules. The molecules are random combinations of domains A, B, C, D, E of protein A of staphylococcus, domains B1, B2, B3, B4, B5 of protein L of digest streptococcus, and domains G1, G2 of protein G of streptococcus. Examples of the molecules include B3-G1-B2-G2-B5, B2-D-G1-B5-C-G1, B5-A-B2-G1-B1, B3-D-B3, B3-D-B3-D-B3, B2-G2-A-B3-D-G1, B1-G1-B3-G1, B2-D-B3-D-B1, B2-C-B4-E-G1, and B2-C-G2-B1-D-G1. Yang et al. (YANG HUA ET AL: "Evolutional selection of a combinatorial phage library displaying randomly-rearranged various single domains of immunoglobulin (Ig)-binding proteins (IBPs) with four kinds of Ig molecules," BMC MICROBIOLOGY, BIOMED CENTRAL LTD. GB, vol. 8, no. 1, page 137, (2008-08-13)) recite two combinations of Ig-binding domains, PA(A)-PG and PA(A)-PL, in which PA(A) is domain A of protein A, PG is domain B2 of protein G, and PL is domain B3 of protein L. WO 2015/005859 A1 recites a polypeptide comprising a mutant of a domain B of protein A (as specified by SEQ ID NO 1), or a domain C of protein A (as specified by SEQ ID NO: 2), and at least the glutamine residue at position 9 of SEQ ID Nos: 1-2 has been mutated to an amino acid other than asparagine. Susanne Gülich et al. (Susanne Gülich ET AL: "Engineering streptococcal protein G for increased alkaline stability," Protein engineering, pages 835-842 (2002-10-01)) recite that the most sensitive amino acid towards alkaline conditions in the C2 domain of protein G is Asn36.

The disclosure aims to overcome the drawbacks in the prior art and provide an immunoglobulin binding protein and a preparation method and an application thereof. As described above, three immunoglobulin binding proteins of protein A, protein G and protein L have different binding types and binding forces to immunoglobulin IgG, IgA, IgM and IgE. One object of the disclosure is to select a structural domain with the strongest binding ability in the three immunoglobulin binding proteins, for example, a Structural domain B of a protein A, a Structural domain C2 of a protein G and a Structural domain B3 of a protein L are taken as templates, on that basis, the expression level of the protein in Escherichia coli is higher and the stability is higher through codon optimization and amino acid mutation.

The first object of the disclosure is to provide an immunoglobulin binding protein. The binding protein is a paired combination of two repeats of a structural domain of protein A, two repeats of a structural domain of protein G, and two repeats of a structural domain of protein L, and the binding protein has an amino acid sequence of SEQ ID NO: 10, SEQ ID NO: 11, or SEQ ID NO: 12; or the binding protein is a combination of two repeats of a structural domain of protein A, two repeats of a structural domain of protein G, and two repeats of a structural domain of protein L, and the binding protein has an amino acid sequence of SEQ ID NO: 13; the structural domain of protein A is a variant of a structural domain B of the protein A, and has an amino acid sequence of SEQ ID NO: 1; the structural domain of the protein G is a variant of a structural domain C2 of the protein G, and has an amino acid sequence of SEQ ID NO: 5; and the structural domain of the protein L is a variant of a structural domain B3 of the protein L, and has an amino acid sequence of SEQ ID NO: 9.

Further, the structural domain of the protein A formed through mutations in an amino acid sequence of a parent molecule of the structural domain B of the protein A, and the mutation sites are N6G, N23T, G29A, and N52K. The structural domain of the protein G is formed through mutations in an amino acid sequence of the parent molecule of the structural domain C2 of the protein G, and the mutation sites are N7T, N34K, N36A and W42K. The structural domain of the protein L is formed through mutations in an amino acid sequence of the parent molecule of the structural domain B3 of the protein L, the mutation sites are N17K, Q26A, A35E, A57V, L59V, G62K, N67T, and R69K.

The alkali-resisting stabilities of the protein A, the protein G and the protein L are improved through gene site-directed mutagenesis. Through gene site-directed mutagenesis, the adsorption capacity of the variant of protein A to the IgG3 subtype is improved.

The second object of the disclosure is to provide a method for preparing an immunoglobulin binding protein. The preparation method comprises the following steps:
(1) combining the two repeats of the structural domain B of the protein A, the two repeats of the structural domain C2 of the protein G, and the two repeats of the structural domain B3 of the protein L to construct a gene recombinant plasmid and an expression strain, and the gene recombinant plasmid comprises a gene capable of encoding the amino acid sequence as shown in SEQ ID NOs: 10-13; and
(2) expressing and purifying the recombinant immunoglobulin binding protein and evaluating the adsorption performance thereof.

Further, the expression strain is escherichia coli BL21(DE3), and the plasmid is PET30a.

In the disclosure, a target gene segment containing a restriction site is directly synthesized, and the gene for coding the target protein is transformed into a vector containing a promoter and a signal sequence. The plasmid is constructed by using the restriction site and is transformed into an Escherichia coli expression strain BL21(DE3), and subsequent protein purification is facilitated by his tagging of the vector. The recombinant Escherichia coli is placed in a fermentation culture medium and cultured at 20-40°C, an inducer IPTG (isopropyl thiogalactoside) is added for induced expression when the OD₆₀₀ value is detected to 0.5-1.0, culturing is continued at 20-40°C for 5-24 h, centrifuging is carried out before the OD600 value is detected to begin to drop, and the bacterial cells are collected.

The third object of the disclosure is to provide a purification method of an immunoglobulin binding protein, and the purification method adopts a three-step chromatography method to purify the protein. The three-step chromatography method comprises the following specific steps: the first step of affinity chromatography, the second step of ion exchange chromatography, and the third step of hydrophobic chromatography; or the first step of hydrophobic chromatography, the second step of affinity chromatography, and the third step of ion exchange chromatography.

Preferably, the method further comprises: replacing a buffer solution with a volume of 5 times that of an ultrafiltration membrane bag between different chromatography steps, and replacing a final protein solution in normal saline, and the pore diameter of the ultrafiltration membrane bag is 5 K.

Preferably, an equilibrium liquid used in the affinity chromatography is 10 to 50 mM bis-tris, and an eluent is 180 mM bis-tris of imidazole.

Preferably, an equilibrium liquid used in the ion exchange chromatography is 10 to 50 mM bis-tris containing 0.05 to 2 wt. % of TritonX-100, a rinsing solution is 10 to 50 mM bis-tris, and the eluent is bis-tris containing 150 mM NaCl. In the ion exchange chromatography of the disclosure, bis-Tris is selected to replace tris-HCl due to the fact that the tris-HCl contains amino, which influences downstream synthetic packing.

Preferably, an equilibrium liquid used in the hydrophobic exchange chromatography is PBS containing 1.5 M (NH₄)₂SO₄, and an eluent is PBS; and the pH values of the equilibrium liquid, the rinsing solution and the eluent are all 7.2. The main purpose of the hydrophobic chromatography is to further purify the protein and remove endotoxin.

Preferably, a packing used in the hydrophobic chromatography is phenyl sepharose FF, Phenyl bestarose FF, Phenyl bestarose HP or other packing with the same properties; a packing used in the affinity chromatography is TALON superflow, Ni sepharose 6FF, Ni Bestarose FF or other packing with the same properties; and a packing used in the ion exchange chromatography is DEAE sepharose FF, capto DEAE or other packing with the same properties.

Due to the problem of falling of heavy metal ions, the service life of metal ion chelation chromatography is generally not more than 20 times, so that the protein purification by affinity chromatography is not considered in industrial production. In recent years, with the continuous improvement of synthetic packing technology, GE has launched an affinity chromatography packing that can purify his-tagged proteins with alkali resistance (pH 2-14) and high binding capacity, such as TALON superflow. In the disclosure, the protein is purified by the three-step chromatography method of metal ion chelation chromatography, ion exchange chromatography and hydrophobic chromatography. Experiments show that the using times of the packing can be effectively improved to over 50 times by using a bis-tris solution for loading and cleaning and using 0.01 M NaOH for cleaning a chromatographic column. The service life of the packing is greatly prolonged, and the packing can be used for industrial production of the protein. The protein in the disclosure is purified on a large scale by the three-step chromatography method, so that the protein quality is further stabilized, and the protein purity is ensured to be more than 97%. The endotoxin level is lower than 1 Eu/mg, and the requirement of clinical protein is met.

Further, the affinity chromatography comprises the following steps: balancing 5 times of column volume by using a solution containing 5-50 mM bis-tris and having the pH of 6.5-7.2, and loading a protein solution; continuing to balance 5 times of column volume by using a solution containing 5-50 mM bis-tris and having the pH of 6.5-7.2; rinsing 1 time of column volume by using 0.01 M NaOH; balancing 5 times of column volume by using a solution containing 5-50 mM bis-tris and having the pH of 6.5-7.2; and finally eluting and collecting the protein by using a bis-tris buffer solution containing 50-800 mM imidazole.

Further, the ion exchange chromatography comprises the following steps: balancing 5 times of column volume by using a solution containing 10-50 mM bis-tris and 0.05-2 wt. % TritonX-100 and having pH of 6.5-7.2, loading a protein solution, and continuously balancing 5 times of column volume by using a solution containing 10-50 mM bis-tris and 0.05-2 wt. % TritonX-100; then rinsing 5 times of column volume by using 10-50 mM bis-tris rinsing solution; and finally eluting and collecting the protein by using a 10-50 mM bis-tris buffer solution containing 100-500 mM NaCl.

Further, the hydrophobic chromatography comprises the following steps: balancing 5 times of column volume by using a PBS containing 1.0 to 1.5 M (NH₄)₂SO₄, and loading a protein solution containing 1.5 M (NH₄)₂SO₄; continuously balancing 5 times of column volume by using the PBS containing 1.5 M (NH₄)₂SO₄; and finally eluting by using the PBS and collecting the protein solution.

Preferably, the PBS comprises the following components: 8.0 mmol/L of disodium hydrogen phosphate, 2.0 mmol/L of monopotassium phosphate and 131 mmol/L of NaCl.

The fourth object of the disclosure is to provide a use of an immunoglobulin binding protein in preparation of an adsorption packing.

Further, the immunoglobulin binding protein of the disclosure is coupled to a solid phase vector to prepare an immunoadsorbent, the immunoadsorbent is washed with 1 M NaOH for more than 6 h or washed with 0.5 M NaOH for more than 24 h or washed with 0.1 M NaOH for more than 72 h, and the decrease rate of the adsorption performance is not less than 10%.

Further, the immunoglobulin binding protein of the disclosure is coupled to the solid phase vector to prepare the immunoadsorbent, and the immunoadsorbent is washed and disinfected with sterile sodium hydroxide or saturated sodium carbonate or saturated sodium bicarbonate solution, to prolong the service life of the adsorption column.

The fifth object of the disclosure is to provide the immunoglobulin binding protein for use in the treatment of autoimmune diseases.

The sixth object of the disclosure is to provide the immunoglobulin binding protein for use in the treatment of clinical autoimmune diseases or other diseases related to Ig in a human body.

The disclosure has the following beneficial effects: the immunoglobulin binding protein of the disclosure comprises the Structural domain B of the protein A, the Structural domain C2 of the protein G and the Structural domain B3 of the protein L at the same time, has certain complementarity through different binding characteristics of the protein A, the protein G and the protein L, and is a multifunctional IBP molecule with wider reactivity and higher affinity. The three-step chromatography method of the immunoglobulin binding protein can effectively increase the number of use times of the packing and prolong the service life of the packing. The three-step chromatography method can stabilize the protein quality, ensure that the protein purity is greater than 97% and the endotoxin level is lower than 1 Eu/mg, and meet the requirements of clinical proteins.

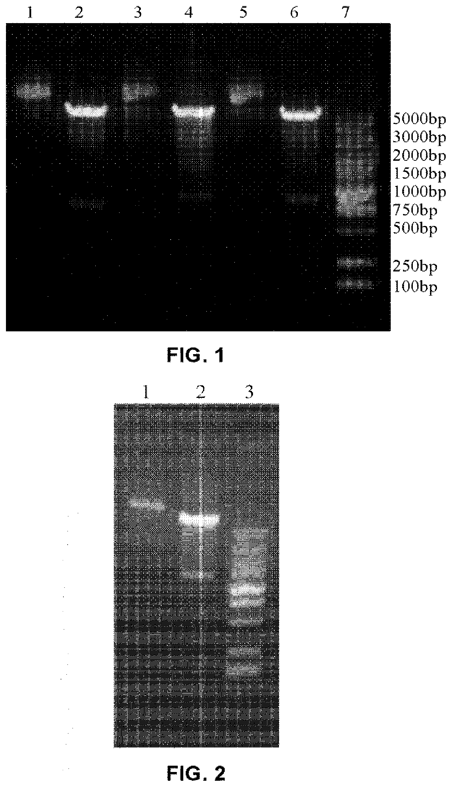

FIG. 1 shows a plasmid diagram of recombinant proteins AG, AL and GL constructed in Example 1 and an SDS-PAGE pattern of a target protein fragment obtained after enzyme digestion, wherein 1 represents a plasmid diagram of the protein AG (PET30a); 2 represents a plasmid diagram of the protein AG after enzyme digestion (708bp); 3 represents a plasmid diagram of the protein AL (PET30a); 4 represents a plasmid diagram of the protein AL after enzyme digestion (810bp); 5 represents a plasmid diagram of the protein GL (PET30a); 6 represents a plasmid diagram of the protein GL after enzyme digestion (804bp); and 7 represents DNA Ladder 5000.
FIG. 2 shows a plasmid diagram of a recombinant protein AGL constructed in Example 1 and an SDS-PAGE pattern of a target protein fragment obtained after enzyme digestion, wherein 1 represents a plasmid diagram of the protein AGL (PET30a); 2 represents a plasmid diagram of the protein AGL after enzyme digestion (1152bp); and 3 represents DNA Ladder 5000.

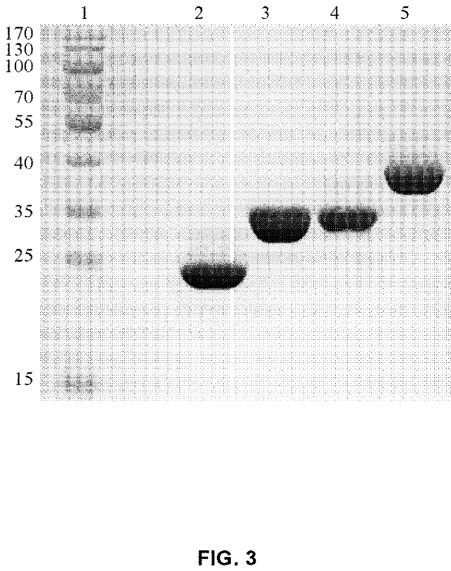

FIG. 3 shows an SDS-PAGE pattern of a supernatant after breaking of a recombinant bacterial cell constructed in the Example 2, wherein 1 represents a protein Marker; 2 represents a protein AG bacterial cell breaking supernatant electrophoresis pattern (24 KD); 3 represents a protein AL bacterial cell breaking supernatant electrophoresis pattern (32 KD); 4 represents a protein GL bacterial cell breaking supernatant electrophoresis pattern (33 KD); and 5 represents a protein AGL bacterial cell breaking supernatant electrophoresis pattern (38 KD).

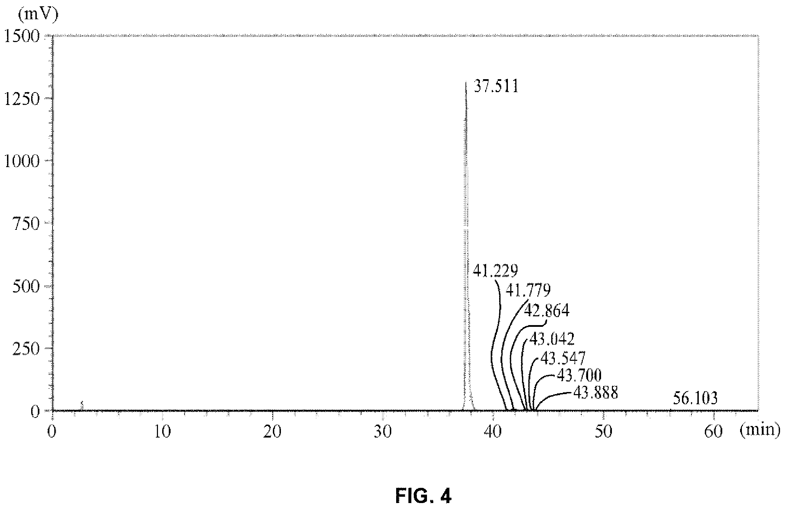

FIG. 4 shows a HPLC pattern of a purified protein AGL.

To illustrate the technical solutions, objects and advantages of the disclosure more concisely and clearly, the disclosure is further described in detail below in conjunction with specific embodiments.

### Example 1 Vector construction and expression of immunoglobulin binding proteins

An amino acid sequence disclosed in SEQ ID No: 1-13 was artificially synthesized, recombinant plasmid PET30a (Novagen) capable of coding the gene sequence of the target amino acid sequence was connected by using restriction sites Ndel and Xhol and then was transformed into a competent Escherichia coli strain BL21(DE3) (merck millipore). 50 mL of LB culture medium was added into a 250 mL triangular flask and cultured with kanamycin with final concentration of 50 µg/mL at 37°C at a speed of 180 rpm for 4 h, IPTG with the final concentration of 50 µg/mL was added to perform induced expression, culturing was performed for 5 h, and then bacterial cell were collected.

### Example 2 Breaking of bacterial cells

1 kg of bacterial cells containing an immunoglobulin binding protein was taken and added into a PBS buffer solution with the pH of 7.2 according to the ratio of mass to volume of 1 kg: 10 L; the bacterial cells and the buffer solution were uniformly stirred and then broken by using a high-pressure homogenizer, breaking was performed twice under the pressure of 800 bar, centrifuging was performed at the temperature of 4°C and a speed of 8,000 rpm for 15 min, and about 9.5 L of supernatant was collected. Hydrochloric acid was added into the obtained solution to adjust the pH to 3.0, centrifuging was performed at the temperature of 4°C and a speed of 8,000 rpm for 15 min, the supernatant was collected, the pH was adjusted to 7.0 by using sodium hydroxide, and centrifuging was performed at a speed of 8,000 rpm for 15 min, thereby obtaining the crushed supernatant of the bacterial cells.

### Example 3 Purification of immunoglobulin binding protein

(1) Affinity chromatography: a chromatographic column packed with a TALON superflow metal ion chelating packing was balanced by 5 times of column volume by using a solution containing 10 mM bis-tris (bis (2-hydroxyethyl) triamino (hydroxymethyl) methane) and having pH value of 7.2; then the broken supernatant obtained in the Example 2 was loaded; the chromatographic column was balanced by 5 times of column volume by using a solution containing 10 mM bis-tris and having pH value of 7.2 after loading; rinsing was performed by 1 time of column volume by using 0.01 M NaOH; then balancing was performed by 5 times of column volume by using a solution containing 10 mM bis-tris and having pH value of 7.2; finally eluting was performed by using a solution containing 180 mM imidazole and having pH value of 7.2; and protein was collected. The main purpose of affinity chromatography was to capture the protein, the protein with weak binding force was washed off by using low-concentration sodium hydroxide during purification through the alkali-resistant characteristics of the metal ion chelating packing and the protein to be purified, the high-purity protein was obtained by a one-step method, and meanwhile, endotoxin can be removed by washing with sodium hydroxide, and the endotoxin content of the protein in an elution peak was reduced.
(2) Ion exchange chromatography: a buffer solution was replaced by a solution containing 10 mM Bis-tris and 1.0 wt. % TritonX-100 through a 5 K ultrafiltration membrane. A chromatographic column packed with a DEAE Bestarose chromatography packing was balanced by 5 times of column volume by using a solution containing 10 mM Bis-tris and 1.0 wt. % TritonX-100 and having pH of 7.2; a protein solution was loaded; after loading, the balancing was continuously performed by 5 times of column volume by using a solution containing 10 mM Bis-tris and 1.0 wt. % TritonX-100 and having pH of 7.2; rinsing was performed by 5 times of column volume by using a rinsing solution containing 10 mM Bis-tris and having pH of 7.2; and eluting was performed by using 10 mM Bis-Tris containing 150 mM/L NaCl and an eluent with pH of 7.2; and an elution peak was collected. In the ion exchange chromatography, bis-Tris was selected to replace tris-HCl due to the fact that the tris-HCl contained amino which influenced downstream synthetic packing.
(3) Hydrophobic chromatography: a buffer solution was replaced by a PBS through a 5 K ultrafiltration membrane. A chromatographic column packed with a Phenyl bestarose FF hydrophobic chromatography packing was balanced by 5 times of column volume by using a PBS solution containing 1.5 M (NH₄)₂SO₄ and having pH of 7.2; the protein solution obtained in the step (2) was diluted by 5 times by using the PBS solution containing 1.5 M (NH₄)₂SO₄ and then was loaded; balancing was performed by 5 times of column volume by using the PBS solution containing 1.5 M (NH₄)₂SO₄ and having pH of 7.2; eluting was performed by using the PBS solution; and an elution peak was collected. The buffer solution was replaced into a normal saline system by using an ultrafiltration system to obtain a target protein solution. The hydrophobic chromatography mainly aimed to further purify and concentrate the protein and remove a small amount of endotoxin.

The endotoxin content of the obtained final protein solution was measured to be 0.4 Eu/mg by using a gel semi-quantitative method-limulus reagent. The protein purity was measured by using a reversed phase HPLC method, and the purity was more than 97 percent, which was shown in FIG. 4.

### Example 4 Method for synthesizing adsorption packing

A method for synthesizing an adsorption packing referred to the patent CN201910353718.1. 100 g of Sepharose 6FF was washed with water for injection of which the volume was about 10 times that of the Sepharose 6FF; 100 mL of 1 M NaOH solution, 30 mL of epoxy bromopropane, 70 mL of 1, 4-butanediol diglycidyl ether and 0.2 g of sodium borohydride were added and stirred and reacted at the temperature of 30°C for 1.5 h; and the reaction product was rinsed with a large amount of water for injection after the reaction was ended, thereby obtaining epoxy activated Sepharose gel. 100 mL of the synthesized epoxy activated Sepharose gel and 150 mL of 0.1 mol/L borate buffer solution were added into a 500 mL reaction container; the pH value of the system was controlled to be 7.5-8.5; 14 g of immunoglobulin binding protein was added and reacted in a 37°C constant-temperature system for 20 h; then the reaction was stopped, the packing was rinsed with water for injection of which the volume was about 10 times that of the packing; and 200 mL of 0.2 M ethanol amine solution was added to seal the unreacted epoxy group and reacted at the temperature of 20°C for 10 h. The reaction product was rinsed with a large amount of water for injection after the reaction was ended, and finally, the reaction product was preserved in a preservative-containing solution.

### Example 5 Test of adsorption performance of protein A and each combined protein synthetic packing on human IgG in the disclosure

0.5 mL of packing synthesized in the Example 4 was added into a 10 mL EP tube; 5 mL of human plasma was added; and the EP tube was placed in a shaking table and slowly shaken up at room temperature for 1 h. After the reaction ended, the reaction solution was poured into a disposable affinity chromatography column and rinsed with about 100 mL of equilibrium liquid (PBS); then eluting was performed with 40 mL of eluent (2.1 g/L of citric acid and 8.0 g/L of NaCl); an elution peak was collected; and the value of OD₂₈₀ was detected by ultraviolet method, wherein the adsorption performance was (mg/mL) = ((OD280/1.38)×40)/0.5. Meanwhile, the content of IgG3 in the eluent was measured by using an IgG3 detection kit, and the content of IgG3 adsorbed by each packing was calculated. The results in the Table 1 showed that except the protein AG, the difference between the adsorption performance of AL, GL and AGL and the adsorption performance of the protein A on the total IgG was not large, but the adsorption ratio of the combined protein on the IgG3 was obviously increased.

**Table 1: Results of adsorption performance of protein A and each combination protein synthetic packing to human IgG in the disclosure**

| Protein name | Static adsorption performance for IgG (mg/mL) | Proportion of IgG3 subtype (%) |
|---|---|---|
| A without mutation (control) | 54 | 2.42 |
| A | 55 | 3.02 |
| AG | 46 | 2.51 |
| AL | 49 | 3.73 |
| GL | 50 | 4.00 |
| AGL | 52 | 4.94 |

### Example 6 Test of alkali resistance of protein AGL synthetic packing

A certain amount of packing prepared by Example 5 was taken and soaked for 1 h, 6 h, 12 h, 24 h and 36 h by using 0.5 M NaOH respectively, then the packing was packed into a disposable affinity chromatography column, and then was washed by using purified water in an amount which was 10 times the volume of the column; and the adsorption performance was determined according to the method of the Example 5, the result was shown in Table 2.

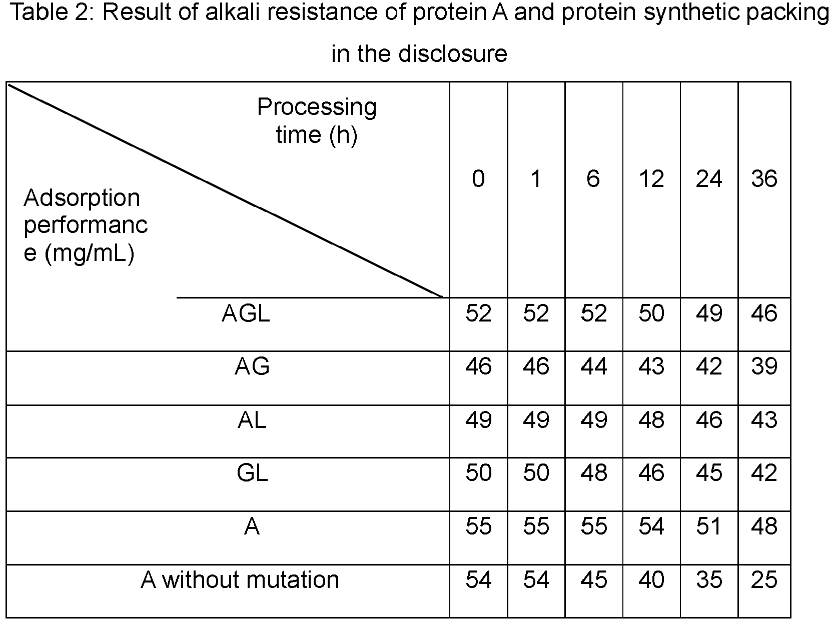

The results showed that the alkali resistance of the mutated protein A packing was remarkably improved compared with that of the protein A packing before mutation. After the protein A packing was treated with 0.5 M NaOH for 36 h, the adsorption performance decline rate was increased from 53.7% to 12.7%. Compared with other proteins, the alkali resistance of protein AG and protein GL was slightly weak, and after the protein AG and protein GL were treated for 36 h, their adsorption performance declined by 15.2% and 16.0% respectively. The stability of the protein A, the protein AGL and the protein AL was equivalent, and their adsorption performance declined by about 12% after treated for 36 h.

## Claims

1. An immunoglobulin binding protein, **characterised in that:**
(1) the binding protein is a paired combination of two repeats of a structural domain of protein A, two repeats of a structural domain of protein G, and two repeats of a structural domain of protein L, and the binding protein has an amino acid sequence of SEQ ID NO: 10, SEQ ID NO: 11, or SEQ ID NO: 12; or
(2) the binding protein is a combination of two repeats of a structural domain of protein A, two repeats of a structural domain of protein G, and two repeats of a structural domain of protein L, and the binding protein has an amino acid sequence of SEQ ID NO: 13;
wherein:
the structural domain of protein A is a variant of a structural domain B of the protein A, and has an amino acid sequence of SEQ ID NO: 1;
the structural domain of the protein G is a variant of a structural domain C2 of the protein G, and has an amino acid sequence of SEQ ID NO: 5; and
the structural domain of the protein L is a variant of a structural domain B3 of the protein L, and has an amino acid sequence of SEQ ID NO: 9.

2. A method for preparing the immunoglobulin binding protein of claim 1, the method comprising:
(1) combining the two repeats of the structural domain B of the protein A, the two repeats of the structural domain C2 of the protein G, and the two repeats of the structural domain B3 of the protein L to construct a gene recombinant plasmid and an expression strain, wherein the gene recombinant plasmid comprises a gene capable of encoding the amino acid sequence as shown in SEQ ID NOs: 10-13; and
(2) expressing and purifying a recombinant immunoglobulin binding protein and evaluating an adsorption performance thereof.

3. A purification method of the immunoglobulin binding protein of claim 1, the method comprising: a first step of affinity chromatography, a second step of ion exchange chromatography, and a third step of hydrophobic chromatography; or a first step of hydrophobic chromatography, a second step of affinity chromatography, and a third step of ion exchange chromatography.

4. The method of claim 3, wherein the method further comprises: replacing a buffer solution with a volume of 5 times that of an ultrafiltration membrane bag between different chromatography steps, and replacing a final protein solution in normal saline; and a pore diameter of the ultrafiltration membrane bag is 5 K.

5. The method of claim 3, wherein an equilibrium liquid used in the affinity chromatography is 10 to 50 mM bis-tris, and an eluent is 180 mM bis-tris of imidazole; an equilibrium liquid used in the ion exchange chromatography is 10 to 50 mM bis-tris containing 0.05 to 2 wt. % of TritonX-100, a rinsing solution is 10 to 50 mM bis-tris, and an eluent is bis-tris containing 150 mM NaCl; an equilibrium liquid used in the hydrophobic exchange chromatography is PBS containing 1.5 M (NH₄)₂SO₄, and an eluent is PBS; and pH values of the equilibrium liquid, the rinsing solution and the eluent are all 7.2.

6. The method of claim 3, wherein a packing used in the hydrophobic chromatography is phenyl sepharose FF, Phenyl bestarose FF, Phenyl bestarose HP or other packing with the same properties; a packing used in the affinity chromatography is TALON superflow, Ni sepharose 6FF, Ni Bestarose FF or other packing with the same properties; and a packing used in the ion exchange chromatography is DEAE sepharose FF, capto DEAE or other packing with the same properties.

7. The method of claim 3, wherein the affinity chromatography comprises the following steps: balancing 5 times of column volume by using a solution containing 5-50 mM bis-tris and having the pH of 6.5-7.2, and loading a protein solution; continuing to balance 5 times of column volume by using a solution containing 5-50 mM bis-tris and having the pH of 6.5-7.2; rinsing 1 time of column volume by using 0.01 M NaOH; balancing 5 times of column volume by using a solution containing 5-50 mM bis-tris and having the pH of 6.5-7.2; and finally eluting and collecting the protein by using a bis-tris buffer solution containing 50-800 mM imidazole.

8. The method of claim 3, wherein the ion exchange chromatography comprises the following steps: balancing 5 times of column volume by using a solution containing 10-50 mM bis-tris and 0.05-2 wt. % TritonX-100 and having pH of 6.5-7.2, loading a protein solution, and continuously balancing 5 times of column volume by using a solution containing 10-50 mM bis-tris and 0.05-2 wt. % TritonX-100; then rinsing 5 times of column volume by using 10-50 mM bis-tris rinsing solution; and finally eluting and collecting the protein by using a 10-50 mM bis-tris buffer solution containing 100-500 mM NaCl.

9. The method of claim 3, wherein the hydrophobic chromatography comprises the following steps: balancing 5 times of column volume by using a PBS containing 1.0 to 1.5 M (NH₄)₂SO₄, and loading a protein solution containing 1.5 M (NH₄)₂SO₄; continuously balancing 5 times of column volume by using the PBS containing 1.5 M (NH₄)₂SO₄; and finally eluting by using the PBS and collecting the protein solution.

10. The method of claim 9, wherein the PBS comprises the following components: 8.0 mmol/L of disodium hydrogen phosphate, 2.0 mmol/L of monopotassium phosphate and 131 mmol/L of NaCl.

11. An adsorption packing comprising the immunoglobulin binding protein of claim 1.

12. The adsorption packing of claim 11, wherein the immunoglobulin binding protein is coupled to a solid phase vector to prepare the adsorption packing; the adsorption packing is washed with 1 M NaOH for more than 6 h or washed with 0.5 M NaOH for more than 24 h or washed with 0.1 M NaOH for more than 72 h, and a decrease rate of an adsorption performance of the adsorption packing is not less than 10%.

13. The adsorption packing of claim 11, wherein the immunoglobulin binding protein is coupled to a solid phase vector to prepare the adsorption packing, and the the adsorption packing is washed and disinfected with sterile sodium hydroxide or saturated sodium carbonate or saturated sodium bicarbonate solution, to prolong the service life of the adsorption packing.

14. The immunoglobulin binding protein of claim 1 for use in the treatment of autoimmune diseases.

15. The immunoglobulin binding protein of claim 1 for use in the treatment of clinical autoimmune diseases or other diseases related to Ig in a human body.

## Patentansprüche

1. Ein Immunglobulin-bindendes Protein, **dadurch gekennzeichnet, dass**:
(1) das Bindungsprotein ist eine Kombination aus zwei Wiederholungen einer Strukturdomäne von Protein A, zwei Wiederholungen einer Strukturdomäne von Protein G und zwei Wiederholungen einer Strukturdomäne von Protein L, und das Bindungsprotein hat eine Aminosäuresequenz von SEQ ID NO: 10, SEQ ID NO: 11 oder SEQ ID NO: 12; oder
(2) das Bindungsprotein ist eine Kombination aus zwei Wiederholungen einer Strukturdomäne von Protein A, zwei Wiederholungen einer Strukturdomäne von Protein G und zwei Wiederholungen einer Strukturdomäne von Protein L, und das Bindungsprotein hat eine Aminosäuresequenz von SEQ ID Nr.: 13;
wobei:
die Strukturdomäne von Protein A ist eine Variante einer Strukturdomäne B von Protein A und weist eine Aminosäuresequenz von SEQ ID Nr.: 1 auf;
die Strukturdomäne des Proteins G ist eine Variante einer Strukturdomäne C2 des Proteins G und weist eine Aminosäuresequenz von SEQ ID Nr.: 5 auf; und
die Strukturdomäne des Proteins L ist eine Variante einer Strukturdomäne B3 des Proteins L und weist die Aminosäuresequenz gemäß SEQ ID Nr. 9 auf.

2. Verfahren zur Herstellung des Immunglobulin-bindenden Proteins nach Anspruch 1, wobei das Verfahren Folgendes umfasst:
(1) Kombinieren der beiden Wiederholungen der Strukturdomäne B des Proteins A, der beiden Wiederholungen der Strukturdomäne C2 des Proteins G und der beiden Wiederholungen der Strukturdomäne B3 des Proteins L, um ein genrekombinantes Plasmid und einen Expressionsstamm zu konstruieren, wobei das Gen rekombinant istDas Plasmid umfasst ein Gen, das in der Lage ist, die in SEQ **ID** Nr.: 10-13 gezeigte Aminosäuresequenz zu kodieren; Und
(2) Exprimieren und Reinigen eines rekombinanten Immunglobulin-bindenden Proteins und Bewerten seiner Adsorptionsleistung.

3. Reinigungsverfahren für das Immunglobulin-bindende Protein nach Anspruch 1, wobei das Verfahren umfasst: einen ersten Schritt der Affinitätschromatographie, einen zweiten Schritt der Ionenaustauschchromatographie und einen dritten Schritt der hydrophoben Chromatographie; oder ein erster Schritt der hydrophoben Chromatographie, ein zweiter Schritt vonAffinitätschromatographie und ein dritter Schritt der Ionenaustauschchromatographie.

4. Verfahren nach Anspruch 3, wobei das Verfahren außerdem Folgendes umfasst: Ersetzen einer Pufferlösung mit einem Volumen, das dem Fünffachen des Volumens eines Ultrafiltrationsmembranbeutels zwischen verschiedenen Chromatographieschritten entspricht, und Ersetzen einer endgültigen Proteinlösung in normaler Kochsalzlösung; und einem Porendurchmesser der UltrafiltrationMembranbeutel ist 5 K.

5. Verfahren nach Anspruch 3, wobei eine bei der Affinitätschromatographie verwendete Gleichgewichtsflüssigkeit 10 bis 50 mM Bis-tris und ein Eluent 180 mM Bis-tris von Imidazol ist; Eine bei der Ionenaustauschchromatographie verwendete Gleichgewichtsflüssigkeit ist 10 bis 50 mM Bistris mit 0,05 bis 2 Gew.-%. % TritonX-100, eine Spüllösung besteht aus 10 bis 50 mM Bis-Tris und ein Eluent ist Bis-Tris mit 150 mM NaCl; eine bei der hydrophoben Austauschchromatographie verwendete Gleichgewichtsflüssigkeit ist PBS mit 1,5 M (NH4)2SO4 und ein Eluent ist PBS; und pH-Werte der Gleichgewichtsflüssigkeit, der SpülungLösung und Eluent sind alle 7.2.

6. Verfahren nach Anspruch 3, wobei eine bei der hydrophoben Chromatographie verwendete Packung Phenylsepharose FF, Phenylbestarose FF, Phenylbestarose HP oder eine andere Packung mit den gleichen Eigenschaften ist; Eine bei der Affinitätschromatographie verwendete Packung ist TALON Superflow, Ni Sepharose 6FF, Ni Bestarose FF oder eine andereVerpackung mit den gleichen Eigenschaften; und eine bei der Ionenaustauschchromatographie verwendete Packung ist DEAE-Sepharose FF, Capto-DEAE oder eine andere Packung mit den gleichen Eigenschaften.

7. Verfahren nach Anspruch 3, wobei die Affinitätschromatographie die folgenden Schritte umfasst: Ausgleichen des 5-fachen Säulenvolumens durch Verwendung einer Lösung, die 5-50 mM Bistris enthält und einen pH-Wert von 6,5-7,2 aufweist, und Beladen mit einer Proteinlösung; weiterhin das Fünffache des Säulenvolumens ausgleichen, indem a verwendet wirdLösung, die 5-50 mM Bis-tris enthält und einen pH-Wert von 6,5-7,2 aufweist; 1-maliges Spülen des Säulenvolumens mit 0,01 M NaOH; Ausgleich des 5-fachen Säulenvolumens durch Verwendung einer Lösung, die 5-50 mM Bis-tris enthält und einen pH-Wert von 6,5-7,2 aufweist; und schließlich Eluieren und Sammeln des Proteins unter Verwendung von Bis-Tris-Pufferlösung mit 50-800 mM Imidazol.

8. Verfahren nach Anspruch 3, wobei die Ionenaustauschchromatographie die folgenden Schritte umfasst: Ausgleich des 5-fachen Säulenvolumens durch Verwendung einer Lösung, die 10-50 mM Bis-tris und 0,05-2 Gew.-% enthält. % TritonX-100 und einem pH-Wert von 6,5-7,2, Laden einer Proteinlösung und kontinuierliches Ausbalancieren des 5-fachen Säulenvolumens unter Verwendung einer Lösung, die 10-50 mM Bistris und 0,05-2 Gew.-% enthält. % TritonX-100; dann Spülen des 5-fachen Säulenvolumens unter Verwendung von 10-50 mM Bis-Tris-Spüllösung; und schließlich Eluieren und Sammeln des Proteins unter Verwendung einer 10-50 mM Bis-Tris-Pufferlösung, die 100-500 mM NaCl enthält.

9. Verfahren nach Anspruch 3, wobei die hydrophobe Chromatographie die folgenden Schritte umfasst: Ausgleichen des 5-fachen Säulenvolumens durch Verwendung von PBS, das 1,0 bis 1,5 M (NH4)2SO4 enthält, und Laden einer Proteinlösung, die 1,5 M (NH4)2SO4 enthält; Kontinuierlicher Ausgleich des 5-fachen Säulenvolumens unter Verwendung desPBS mit 1,5 M (NH4)2SO4; und schließlich Eluieren unter Verwendung von PBS und Sammeln der Proteinlösung.

10. Verfahren nach Anspruch 9, wobei das PBS die folgenden Komponenten umfasst: 8,0 mmol/L Dinatriumhydrogenphosphat, 2,0 mmol/L Monokaliumphosphat und 131 mmol/L NaCl.

11. Adsorptionspackung, umfassend das Immunglobulin-bindende Protein nach Anspruch 1.

12. Adsorptionspackung nach Anspruch 11, wobei das Immunglobulin-bindende Protein an einen Festphasenvektor gekoppelt ist, um die Adsorptionspackung herzustellen; die Adsorptionspackung wird mit 1 M NaOH für mehr als 6 Stunden oder mit 0,5 M NaOH für mehr als 24 Stunden gewaschen oder mit 0,1 M NaOH für mehr als 24 Stunden gewaschen72 Stunden und die Abnahmerate der Adsorptionsleistung der Adsorptionspackung beträgt nicht weniger als 10 %.

13. Adsorptionspackung nach Anspruch 11, wobei das Immunglobulin-bindende Protein an einen Festphasenvektor gekoppelt ist, um die Adsorptionspackung herzustellen, und die Adsorptionspackung mit sterilem Natriumhydroxid oder gesättigtem Natriumcarbonat oder gesättigtem Natriumbicarbonat gewaschen und desinfiziert wirdLösung, um die Lebensdauer der Adsorptionspackung zu verlängern.

14. Das Immunglobulin-bindende Protein nach Anspruch 1 zur Verwendung bei der Behandlung von Autoimmunerkrankungen.

15. Das Immunglobulin-bindende Protein nach Anspruch 1 zur Verwendung bei der Behandlung klinischer Autoimmunerkrankungen oder anderer mit Ig in Zusammenhang stehender Erkrankungen im menschlichen Körper.

## Revendications

1. Protéine de liaison à l'immunoglobuline, **caractérisée en ce que** :
(1) la protéine de liaison est une combinaison de deux répétitions du domaine structurel de la protéine A, de deux répétitions du domaine structurel de la protéine G, de deux répétitions du domaine structurel de la protéine L, et la protéine de liaison a une séquence d'acides aminés de SEQ ID NO : 10, SEQ ID NO : 11, ou SEQ ID NO : 12 ; ou
(2) la protéine de liaison est une combinaison de deux répétitions du domaine structurel de la protéine A, de deux répétitions du domaine structurel de la protéine G et de deux répétitions du domaine structurel de la protéine L, et la protéine de liaison a une séquence d'acides aminés de SEQ ID NO : 13 ;
dans lequel :
le domaine structurel de la protéine A est une variante du domaine structurel B de la protéine A, et possède une séquence d'acides aminés de SEQ ID NO : 1;
le domaine structurel de la protéine G est une variante du domaine structurel C2 de la protéine G, et possède une séquence d'acides aminés de SEQ ID NO : 5 ; et
le domaine structurel de la protéine L est une variante du domaine structurel B3 de la protéine L, et possède une séquence d'acides aminés de SEQ ID NO: 9.

2. Procédé de préparation de la protéine de liaison à l'immunoglobuline selon la revendication 1, le procédé comprend :
(1) la combinaison des deux répétitions du domaine structurel B de la protéine A, des deux répétitions du domaine structurel C2 de la protéine G et des deux répétitions du domaine structurel B3 de la protéine L pour construire un plasmide recombinant et une souche d'expression, dans lequel le plasmide recombinant comprend un gène capable de coder la séquence d'acides aminés telle que représentée dans les SEQ ID Nos : 10-13 ; et
(2) l'expression et la purification d'une protéine recombinante de liaison à l'immunoglobuline et l'évaluation de ses performances d'adsorption.

3. Procédé de purification de la protéine de liaison à l'immunoglobuline de la revendication 1, le procédé comprend : une première étape de chromatographie d'affinité, une deuxième étape de chromatographie d'échange d'ions et une troisième étape de chromatographie hydrophobe ; ou une première étape de chromatographie hydrophobe, une deuxième étape de chromatographie d'affinité et une troisième tape de chromatographie d'échange d'ions.

4. Procédé de la revendication 3, dans lequel le procédé comprend en outre : le remplacement d'une solution tampon par un volume 5 fois supérieur à celui d'un sac à membrane d'ultrafiltration entre différentes étapes de chromatographie, et le remplacement d'une solution protéique finale dans une solution saline normale ; le diamètre des pores du sac à membrane d'ultrafiltration est de 5 K.

5. Procédé de la revendication 3, dans lequel un liquide d'équilibre utilisé dans la chromatographie d'affinité est du bis-tris 10 à 50 mM, et un éluant est du bis-tris 180 mM d'imidazole ; un liquide d'équilibre utilisé dans la chromatographie d'échange d'ions est du bis-tris 10 à 50 mM contenant 0,05 à 2 % en poids de TritonX-100, une solution de rinçage est du bis-tris 10 à 50 mM, un éluant est du bis-tris contenant 150 mM de NaCl (chlorure de sodium); le liquide d'équilibre utilisé dans la chromatographie d'échange hydrophobe est du PBS, contenant 1,5 M de (NH₄)₂SO₄, et l'éluant est du PBS ; les valeurs de pH du liquide d'équilibre, de la solution de rinçage et de l'éluant sont toutes de 7,2.

6. Procédé de la revendication 3, dans lequel le garnissage utilisé dans la chromatographie hydrophobe est du phényl sepharose FF, du phényl bestarose FF, Phenyl bestarose HP ou tout autre garnissage présentant les mêmes propriétés ; le garnissage utilisé dans la chromatographie d'affinité est du TALON superflow, du Ni sepharose 6FF, du Ni Bestarose FF ou tout autre garnissage présentant les mêmes propriétés ; un garnissage utilisé dans la chromatographie d'échange d'ions est du DEAE sepharose FF, du capto DEAE ou tout autre garnissage présentant les mêmes propriétés.

7. Procédé de la revendication 3, dans lequel la chromatographie d'affinité comprend les étapes suivantes : équilibrer 5 fois le volume de la colonne à l'aide d'une solution contenant 5 à 50 mM de bis-tris, et un pH de 6,5 à 7,2, charger une solution protéique ; continuer à équilibrer 5 fois le volume de la colonne à l'aide d'une solution contenant 5 à 50 mM de bis-tris, avec un pH de 6,5 à 7,2 ; rinçage d'une fois du volume de la colonne à l'aide de NaOH 0,01 M ; équilibrer 5 fois le volume de la colonne à l'aide d'une solution contenant 5 à 50 mM de bis-tris, et un pH de 6,5 à 7,2 ; et enfin éluer et recueillir la protéine à l'aide d'une solution tampon bis-tris contenant 50 à 800 mM d'imidazole.

8. Procédé de la revendication 3, dans lequel la chromatographie d'échange d'ions comprend les étapes suivantes : équilibrer 5 fois le volume de la colonne à l'aide d'une solution contenant 10 à 50 mM de bis-tris et 0,05 à 2 % en poids de TritonX-100, avec un pH de 6,5 à 7,2 ; charger une solution de protéines et équilibrer en continu 5 fois le volume de la colonne à l'aide d'une solution contenant 10 à 50 mM de bis-tris ayant un pH de 6,5 à 7,2. % de TritonX-100 et un pH de 6,5 à 7,2 ; charger une solution de protéines, et équilibrer en continu 5 fois le volume de la colonne à l'aide d'une solution contenant 10 à 50 mM de bis-tris et 0,05 à 2 % en poids de TritonX-100 ; puis rincer 5 fois le volume de la colonne à l'aide d'une solution de rinçage de 10 à 50 mM de bis-tris ; et enfin, éluer et recueillir la protéine à l'aide d'une solution tampon de 10 à 50 mM de bis-tris contenant 100 à 500 mM de NaCl.

9. Procédé de la revendication 3, dans lequel la chromatographie hydrophobe comprend les étapes suivantes : équilibrer 5 fois le volume de la colonne en utilisant un PBS contenant 1,0 à 1,5 M de (NH₄)₂SO₄, et charger une solution protéique contenant 1,5 M de (NH₄)₂SO₄ ; équilibrer en continu 5 fois le volume de la colonne à l'aide du PBS contenant 1,5 M (NH₄)₂SO₄ ; et enfin éluer à l'aide du PBS et recueillir la solution protéique.

10. Procédé de la revendication 9, dans lequel les composants de PBS sont les suivants : 8,0 mmol/L d'hydrogénophosphate disodique, 2,0 mmol/L de phosphate monopotassique et 131 mmol/L de NaCl.

11. Garnissage adsorbant comprenant la protéine de liaison à l'immunoglobuline de la revendication 1.

12. Le garnissage adsorbant de la revendication 11, dans lequel la protéine de liaison à l'immunoglobuline est couplée à un vecteur en phase solide pour préparer le garnissage adsorbant ; le garnissage adsorbant est lavé avec du NaOH 1 M pendant plus de 6 heures ou lavé avec du NaOH 0,5 M pendant plus de 24 heures ou lavé avec du NaOH 0,1 M pendant plus de 72 heures, et le taux de diminution des performances d'adsorption du garnissage adsorbant n'est pas inférieur à 10 %.

13. Le garnissage d'adsorption de la revendication 11, dans lequel la protéine de liaison à l'immunoglobuline est couplée à un vecteur en phase solide pour préparer le garnissage d'adsorption ; le garnissage d'adsorption est lavé et désinfecté avec de l'hydroxyde de sodium stérile ou une solution saturée de carbonate de sodium ou de bicarbonate de sodium, afin de prolonger la durée de vie du garnissage d'adsorption.

14. Protéine de liaison à l'immunoglobuline de la revendication 1, destinée à être utilisée dans le traitement de maladies auto-immunes.

15. Protéine de liaison à l'immunoglobuline de la revendication 1, destinée à être utilisée dans le traitement de maladies auto-immunes cliniques ou d'autres maladies liées à l'Ig dans le corps humain.
